(19) **Europäisches Patentamt / European Patent Office / Office européen des brevets**

(11) **EP 2 959 311 B1**

(12) **FASCICULE DE BREVET EUROPEEN**

(45) Date de publication et mention
de la délivrance du brevet:
**27.06.2018 Bulletin 2018/26**

(21) Numéro de dépôt: **14705146.0**

(22) Date de dépôt: **17.02.2014**

(51) Int Cl.:
**G01S 7/52** (2006.01)  **A61B 8/08** (2006.01)
**A61B 5/02** (2006.01)  **A61B 5/021** (2006.01)

(86) Numéro de dépôt international:
**PCT/EP2014/053030**

(87) Numéro de publication internationale:
**WO 2014/128084 (28.08.2014 Gazette 2014/35)**

(54) **PROCÉDÉ DE GÉNÉRATION D'UNE IMAGE D'ÉLASTICITÉ**

VERFAHREN ZUR ERZEUGUNG EINES ELASTIZITÄTSBILDES

METHOD FOR GENERATING AN ELASTICITY IMAGE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **22.02.2013 FR 1351549**

(43) Date de publication de la demande:
**30.12.2015 Bulletin 2015/53**

(73) Titulaires:
• **Université Grenoble Alpes**
**38400 Saint-Martin-d'Hères (FR)**
• **VAL-CHUM, SOCIÉTÉ EN COMMANDITE**
**Montréal, QC H3V 1H8 (CA)**

(72) Inventeurs:
• **OHAYON, Jacques**
**73100 Tresserve (FR)**
• **DELEAVAL, Flavien**
**73800 Laissaud (FR)**
• **CLOUTIER, Guy**
**Repentigny, Québec J5Z 4J6 (CA)**

(74) Mandataire: **Novagraaf Technologies**
**Bâtiment O2**
**2, rue Sarah Bernhardt**
**CS90017**
**92665 Asnières-sur-Seine Cedex (FR)**

(56) Documents cités:
**EP-A1- 0 908 137    EP-A2- 1 475 040**

• **CESPEDES E I ET AL: "Intraluminal ultrasonic palpation: assessment of local and cross-sectional tissue stiffness", ULTRASOUND IN MEDICINE AND BIOLOGY, NEW YORK, NY, US, vol. 26, no. 3, mars 2000 (2000-03), pages 385-396, XP004295526, ISSN: 0301-5629, DOI: 10.1016/S0301-5629(99)00169-6**
• **FLAVIEN DELEAVAL ET AL: "The Intravascular Ultrasound Elasticity-Palpography Technique Revisited: A Reliable Tool for the In Vivo Detection of Vulnerable Coronary Atherosclerotic Plaques", ULTRASOUND IN MEDICINE & BIOLOGY, vol. 39, no. 8, août 2013 (2013-08), pages 1469-1481, XP055084861, ISSN: 0301-5629, DOI: 10.1016/j.ultrasmedbio.2013.03.001**

**Description**

## DOMAINE TECHNIQUE

La présente invention concerne le domaine technique général de la palpographie de rigidité. Plus précisément, la présente invention concerne un procédé de génération d'une image d'élasticité de module d'Young d'un milieu composite entourant une cavité.

La présente invention peut trouver de nombreuses applications. Elle peut s'appliquer notamment à l'estimation d'un risque de rupture d'une plaque d'athérome.

## PRESENTATION GENERALE DE L'ART ANTERIEUR

L'athérome ou athérosclérose correspond à un remaniement de l'intima des artères de gros et moyen calibre (aorte, artères coronaires, artères cérébrales, artères des membres inférieurs, etc.) par accumulation segmentaire de lipides, glucides complexes, sang et produits sanguins, tissus adipeux, dépôts calcaires et autres minéraux.

Cette pathologie vasculaire est généralement à progression lente (sur des décennies). Elle peut se stabiliser et ne pas présenter de danger notable pour le patient. Mais elle peut aussi dégénérer en une forme instable menant à une rupture de la plaque et, en quelques jours, provoquer des accidents cardiovasculaires ou cérébraux (ACV), mortels ou morbides.

En effet, la rupture d'une plaque met son contenu en contact avec le sang véhiculé par l'artère, ce qui peut entraîner la formation d'un thrombus. Celui-ci perturbe la circulation sanguine dans l'artère atteinte. Il peut aussi se détacher et être transporté par le flux sanguin, et, dans les cas les plus sévères, bloquer complètement la lumière de l'artère, stopper l'irrigation de la région post lésion et provoquer son ischémie.

La caractérisation des tissus présente un intérêt fondamental en diagnostic médical, notamment pour l'estimation d'un risque de rupture d'une plaque d'athérome. Depuis les vingt dernières années, une nouvelle méthode d'imagerie médicale a été développée : il s'agit de l'élastographie ultrasonore.

Basée sur les mêmes principes que la palpation, l'élastographie étudie localement le comportement élastique d'un milieu sous l'action d'une contrainte. Cette étude repose sur l'analyse de signaux ultrasonores radiofréquences acquis avant et après application d'une contrainte, ou acquis pour différents niveaux de contrainte.

Comme indiqué ci-dessus, la plaque d'athérosclérose, impliquent le dépôt de lipides et/ou de collagène sur les parois vasculaires. Ce dépôt résulte en une augmentation ou une diminution de l'élasticité des parois vasculaire. L'élastographie ultrasonore fournit au praticien une information lui permettant d'estimer les risques de rupture d'une plaque d'athérome.

Le document EP 0 908 137 décrit une méthode d'élastographie ultrasonore permettant de fournir une image des caractéristiques élastiques du milieu. Plus précisément, cette méthode permet de déterminer et d'afficher une « raideur » apparente locale de la couche épaisse endoluminal d'une cavité d'un corps tel qu'une artère. Toutefois, cette méthode présente des inconvénients majeures mettant en cause les crédibilités (i.e. ce ne sont pas des élasticités réelles) des formulations d'élasticité locale et globale décrites respectivement dans le document EP 0 908 137.

Notamment, l'élasticité locale de la couche épaisse endoluminale de la cavité du corps est estimée en supposant que :

- les parois interne et externe de celui-ci sont cylindriques et concentriques (i.e. épaisseur uniforme du corps entre les parois cylindriques interne et externe de la cavité),
- les deux parois interne et externe de celui-ci sont soumises à des distributions spatiales de pressions uniformes,

de sorte que les valeurs d'élasticité fournies en mettant en oeuvre cette méthode sont très différentes des valeurs réelles d'élasticité du corps analysé et n'ont ainsi aucun sens physique (i.e. ce n'est pas une élasticité réelle).

Ainsi, la méthode décrite dans EP 0 908 137 ne permet pas au praticien de disposer d'une information suffisamment précise pour établir un diagnostic.

Un but de la présente invention est de proposer un procédé d'élastographie ultrasonore permettant de pallier les inconvénients de la méthode décrite dans EP 0 908 137.

## PRESENTATION DE L'INVENTION

A cet effet, l'invention propose un procédé de traitement d'image permettant la formation d'une image d'élasticité d'un corps incluant une cavité en fonction du ou des matériaux constituant ledit corps, caractérisé en ce que le procédé comprend les étapes consistant à :

- Recevoir une image de déformation illustrant un champ de déplacement des points du corps en fonction d'une différence de pression dans le corps,
- Estimer une fonction de forme du corps à partir de l'image de déformation,
- Calculer une image d'élasticité du corps en fonction de la fonction de forme, de la différence de pression et de l'image de déformation.

Le fait d'estimer une fonction de forme du corps et d'utiliser cette fonction de forme dans le calcul de l'image d'élasticité permet de prendre en compte de la géométrie du corps dans le calcul de l'image d'élasticité. Il est ainsi possible d'obtenir une image d'élasticité cohérente, même pour des corps à la géométrie complexe.

Des aspects préférés mais non limitatifs du procédé selon l'invention sont les suivants :

- l'étape consistant à estimer la fonction de forme comprend :

   ◦ la détection des contours intérieur et extérieur du corps à partir de l'image de déformation pour obtenir une image de contour,
   ◦ l'attribution d'une distribution homogène de l'élasticité dans l'image de contour pour obtenir une image de travail,
   ◦ la détermination de la fonction de forme à partir de l'image de travail ;

- l'étape consistant à estimer la fonction de forme comprend la mise en oeuvre d'une analyse par éléments finis ;
- l'étape de calcul d'une image d'élasticité consiste à calculer une image d'élasticité projetée sur la paroi interne du corps pour obtenir une image d'élasticité projetée du corps ;
- le procédé comprend en outre une étape consistant à superposer l'image d'élasticité projetée du corps sur une image du corps acquise en utilisant un dispositif ultrasonore ;
- le procédé comprend en outre une étape consistant à recevoir un domaine de palpographie correspondant à la sélection par un utilisateur d'une zone du corps que l'utilisateur souhaite étudier, l'étape consistant à calculer une image d'élasticité du corps étant mise en oeuvre sur le domaine de palpographie ;
- l'étape consistant à calculer une image d'élasticité comprend la résolution de l'équation suivante :

$$E_{palpo}^{\text{revisited}}(\theta) = \frac{3}{2} \frac{\left| \int_{R_i(\theta)}^{R_p(\theta)} h*(r,\theta)\, dr \right|}{\left| \int_{R_i(\theta)}^{R_p(\theta)} \varepsilon_{rr}(r,\theta)\, dr \right|} \Delta P$$

Avec :

   ◦ $\theta$, la position angulaire dans le corps,
   ◦ r, la position radiale dans le corps,
   ◦ $\Delta P$ la différence de pression
   ◦ $R_i(\theta)$ et $R_p(\theta)$ les rayons interne et externe d'un domaine de palpographie en fonction de la position angulaire dans le corps
   ◦ $h*(r,\theta)$ la fonction de forme approximée du corps en fonction des positions radiale et angulaire dans le corps,
   ◦ $\varepsilon_{rr}(r,\theta)$ est la déformation radiale réelle du corps en fonction de la position dans le corps.

L'invention concerne également un produit programme d'ordinateur comportant un code programme enregistré sur un support de données lisible par un ordinateur pour exécuter le procédé décrit ci-dessus lorsque le programme d'ordinateur est appliqué à un ordinateur pour être exécuté.

## PRESENTATION DES FIGURES

D'autres caractéristiques et avantages de l'invention ressortiront encore de la description qui suit, laquelle est purement illustrative et non limitative et doit être lue en regard des dessins annexés, sur lesquels :

- la figure 1 illustre des étapes du procédé proposé de calcul d'élasticité d'une plaque d'athérome,
- la figure 2 illustre la plaque d'athérome,
- la figure 3 illustre la couche épaisse endoluminale de la cavité du corps,

- la figure 4 illustre un graphique d'élasticités calculées d'une plaque en fonction de la position angulaire sur la plaque et montre qu'avec notre approche on retrouve bien l'élasticité réelle.

## DESCRIPTION DE MODES DE REALISATION DE L'INVENTION

On va maintenant décrire plus en détail un exemple de procédé applicable à un corps incluant une cavité, et permettant de fournir - en temps réel - une information relative à l'élasticité du corps au niveau de la paroi interne de sa cavité.

Dans la suite, le procédé sera décrit en référence à l'étude de vaisseaux sanguins. Toutefois, il est bien évident à l'homme du métier que ce procédé peut être appliqué à d'autres types de corps incluant une cavité, tel que le coeur ou toutes structures industrielles contenant une cavité et ou la déformation est quantifiée autour de cette cavité.

Dans le cas d'une plaque d'athérome, le procédé comprend généralement les étapes de :

- réception d'une image de déformation illustrant un champ de déplacement ou de déformation des points de la plaque,
- réception d'une différence de pression dans la plaque,

   - estimation d'une fonction de forme de la plaque en utilisant l'image de déformation et la différence de pression,
   - calcule d'une image d'élasticité de la plaque en fonction de la fonction de forme, de la différence de pression et de l'image de déformation.

Ces différentes étapes sont décrites plus en détail dans la suite.

### 1. Etapes du procédé

#### 1.1. Réception d'une image de cartographie de déformation/déplacement des points constituant le corps comprenant une cavité

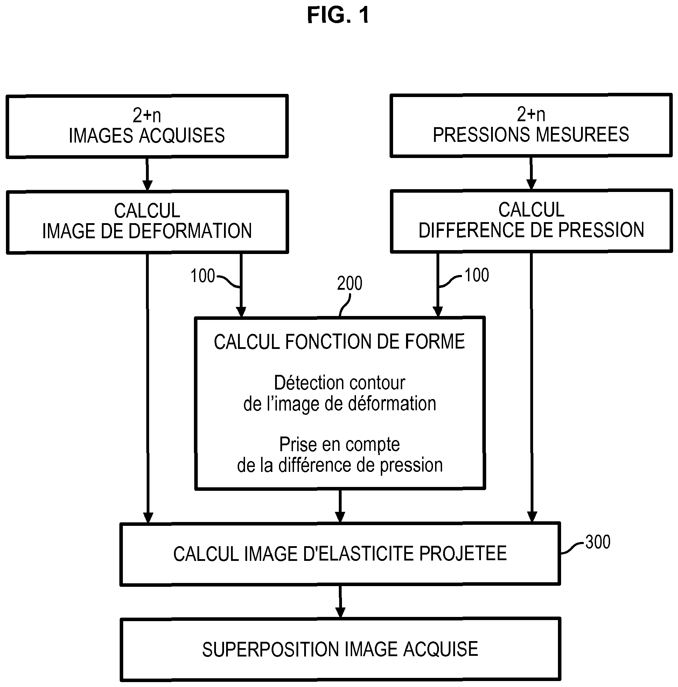

En référence à la figure 1, le procédé comprend une étape de réception 100 d'une image de déformation - dite « élastogramme » - illustrant un champ de déplacement ou de déformation des points du corps en fonction d'une différence de pression dans le corps.

L'élastogramme représente les déformations internes qui résultent de la compression du corps analysé - ici un tissu vasculaire sanguin - en fonction de la pression sanguine. L'élastogramme peut être obtenu par toute méthode connue de l'homme du métier.

Par exemple, l'élastogramme peut être obtenu de la façon suivante. Une sonde ultrasonore est introduite dans une artère d'un patient au niveau d'une portion d'artère que l'on souhaite analyser. Une séquence d'images ultrasonore de la portion de l'artère est acquise pendant que le tissu artériel se comprime/dilate sous l'effet de la pulsation cardiaque. L'élastogramme est obtenu en étudiant la cinétique de la séquence temporelle d'images ultrasonore.

Notamment, l'étude cinétique peut comprendre les étapes consistant à :

- comparer deux images différentes de la portion d'artère acquises à deux instants distincts d'un cycle cardiaque, lesdites images différentes étant acquises pour deux contraintes différentes exercées sur la portion d'artère,
- déterminer une carte des déplacements en comparant la position de chacun des points ou pixels entre les deux images différentes,
- déterminer une différence de pression artérielle entre les deux images différentes en soustrayant les pressions artérielles mesurées lors de l'acquisition desdites images différentes.

On obtient ainsi une image de déformations - ou élastogramme - de la portion d'artère d'une part, et la différence de pression ayant permis d'obtenir cette image de déformation d'autre part.

Ces deux informations (i.e. cartographie des déplacements/déformations du corps analysé, et différence de pression associées à la cartographie des déplacements) sont utiles à la mise en oeuvre d'une étape ultérieure de détermination de l'élasticité de la paroi interne de la cavité du corps.

#### 1.2. Détermination des contours intérieur et extérieur du corps

Le procédé comprend également une étape de détection des contours intérieur et extérieur de la portion d'artère, et plus précisément de l'image de déformation.

La détection des contours du corps incluant la cavité permet de réduire de manière significative la quantité de données contenue dans l'image et élimine les informations non pertinentes pour la mise en oeuvre du procédé, tout en préservant les propriétés structurelles importantes de l'image.

L'étape de détection des contours intérieur et extérieur de l'image peut être basée sur toute technique connue de l'homme du métier. Elle peut par exemple être basée sur un algorithme de recherche d'extremums d'une fonction d'énergie calculée à partir de caractéristiques des images décrivant l'objet à détecter telles que des informations d'arêtes, de contours ou de gradient; des attributs de textures; des distributions de tons gris ou toutes autres fonctions des tons de gris ou des couleurs des images; des information temporelles (flux optique, corrélation ou autres); et des informations a priori sur l'apparence ou la forme du corps à détecter.

L'étape de détection de contour permet d'obtenir une image de contour du corps représentant le contour intérieur (i.e. la lumière) de la portion d'artère, et le contour extérieur de la portion d'artère.

### 1.3. Détermination d'une fonction de forme

Le procédé comprend également une étape de détermination 200 d'une fonction de forme de la portion d'artère. Cette étape de détermination de la fonction de forme est mise en oeuvre à partir :

- de l'image de contour obtenue lors de la mise en oeuvre de l'étape de détection de contour, et
- en supposant une distribution homogène de l'élasticité entre les parois interne et externe de l'image de contour.

L'étape de détermination d'une fonction de forme approchée consiste à trouver une fonction « h* » décrivant au mieux possible la forme réelle (décrite par la fonction « h » que l'on cherche à approcher) de la portion d'artère représentée dans l'image de contour. De préférence, la fonction de forme « h* » est déterminée en utilisant une méthode des éléments finis connue de l'homme du métier :
*Etant donnée une fonction inconnue h(M) que l'on cherche à évaluer en tout point M, l'approximation va consister à trouver une fonction h* s'approchant au mieux de h. L'approximation h* est construite dans une base que l'on choisit, cette base d'approximation est de préférence polynômiale dans le cas des éléments finis. Le nombre de termes de la base, noté n, donne le nombre de points MI pour lesquels l'approximation h* est égale à la fonction h :*

$$\forall \ I \ de \ 1 \ à \ n, \ h^*(MI) = h(MI).$$

### 1.4. Détermination de la cartographie d'élasticité

Le procédé comprend également une étape consistant à estimer 300 l'élasticité du corps, notamment l'élasticité du corps projetée sur la paroi intérieure de la portion d'artère. Cette élasticité projetée est estimée à partir :

- de l'image des déformations - ou élastogramme - du corps,
- de la différence de pression luminale $\Delta P$ entre les images ayant permis d'obtenir l'image des déformations, et
- de la fonction de forme $h^*$ estimée pour le corps.

Ainsi et contrairement à la méthode décrite dans le document EP 0 908 137, le procédé selon l'invention tient compte de la forme de la cavité pour fournir une image des caractéristiques élastiques du corps.

Ceci permet d'obtenir une image d'élasticité dont les valeurs sont plus proches des valeurs d'élasticité réelles. Ceci permet également de limiter les risques de détection de faux négatifs. En effet, avec le procédé décrit dans EP 0 908 137, un grand nombre de faux négatifs sont détectés, ce qui rend l'outil de détection d'un risque de rupture d'une plaque d'athérome inexploitable pour l'utilisateur.

### 1.5. Principe de fonctionnement

Le procédé selon l'invention permet de fournir, en temps réel, une information sur l'élasticité d'une plaque d'athérome.

Cette information permet à un utilisateur de prédire un risque de rupture de la plaque et de définir s'il est nécessaire ou non d'effectuer une étude approfondie de la structure de cette plaque d'athérome.

Le principe de fonctionnement est le suivant. L'utilisateur insère une sonde ultrasonore dans l'artère d'un patient. Pour une portion d'artère donnée, l'utilisateur sélectionne une région d'intérêt autour de la sonde : cette région d'intérêt définit un domaine de palpographie (qui peut correspondre à toute la section de l'artère ou être inclus dans celle-ci). Notons que ce domaine de palpographie est la couche épaisse endoluminale de la cavité du corps et est noté $\Omega_{palpo}$.

Des images ultrasonores sont acquises en utilisant la sonde. La pression sanguine à l'intérieur de l'artère est mesurée pour chaque image acquise.

A partir de deux de ces images, une image de déformation est calculée ainsi qu'une différence de pression correspondant à la différence entre les pressions sanguines ΔP associées aux deux images.

Une fonction de forme est également estimée à partir de l'une des images acquises ou de l'image de déformation. La fonction de forme h* est estimée en détectant les contours intérieur et extérieur de la plaque dans l'image considérée, et en effectuant une analyse en éléments finis.

L'image de déformation, la différence de pression et la fonction de forme sont utilisées pour calculer une image d'élasticité projetée sur la paroi intérieure de la portion d'artère. Cette image d'élasticité est par exemple superposée à une image ultrasonore acquise en utilisant la sonde, les différentes valeurs d'élasticité étant représentées à l'aide d'un code couleur afin de permettre à l'utilisateur d'établir rapidement si la portion d'artère étudiée est saine, ou si la portion d'artère étudiée est à risque et nécessite la mise en oeuvre d'examens complémentaires.

On va maintenant décrire plus en détail certains aspects théoriques de l'invention.

### 2. *Théorie relative au procédé selon l'invention*

Il existe des méthodes de caractérisation *in-vivo* des plaques d'athéroscléroses coronariennes (ou plaque d'athérome) et de la prédiction de leur rupture spontanée basées sur l'élastographie ultrasonore. Toutefois, ces méthodes ne permettent pas une détermination du module d'Young de la plaque en temps réel. En effet, ces méthodes utilisent des algorithmes complexes basés sur des outils d'optimisation mathématique itérative et non linéaire dans le domaine de la mécanique des milieux continus.

EP 0 908 137 décrit une méthode permettant de déterminer et d'afficher en temps réel une raideur apparente locale de la plaque d'athérome.

Toutefois, cette méthode ne tient pas compte de la géométrie complexe de la plaque d'athérome lors de la détermination d'une raideur apparente locale. En effet, il est supposé dans le cas de EP 0 908 137 que :

- la plaque d'athérome est de forme cylindrique,
- la plaque d'athérome est constituée d'un milieu incompressible isotrope homogène et
- la plaque d'athérome est soumise à une distribution uniforme des contraintes radiales externes.

Ces hypothèses ne permettent pas l'obtention d'une information exploitable par l'utilisateur des propriétés mécaniques de la plaque pour estimer un risque de rupture de la plaque.

Le procédé selon l'invention permet de remédier aux inconvénients de la méthode décrite dans EP 0 908 137 en prenant en compte à la fois grâce à l'utilisation d'une fonction de forme :

- la géométrie de la plaque, et
- la géométrie du domaine de palpographie correspondant à une zone d'intérêt sélectionnée par un utilisateur,

lors de l'étape de calcul de l'image d'élasticité de la plaque.

Les défauts de la méthode décrite dans EP 0 908 137 ont été résolus sur la base de la réflexion des inventeurs décrite ci-dessus :

*Sur la base de la relation (1) entre la contrainte déviatorique radiale* $\sigma_{rr}^{dev}(r,\theta)$ *, le module d'Young $E(r,\theta)$ et la composante de déformation radiale $\varepsilon_{rr}(r,\theta)$ pour milieu continu élastique linéaire, incompressible, isotrope, hétérogène, et linéaire :*

$$\sigma_{rr}^{dev}(r,\theta) = \frac{2}{3} E(r,\theta)\, \varepsilon_{rr}(r,\theta)$$

$$(1)$$

*Ici $(r, \theta)$ est le système de coordonnées polaires référée au centre de gravité de la cavité à l'intérieur de laquelle circule le sang.*

*Le module de contrainte-déformation* $E_{palpo}^{new}(\theta)$ *a été redéfinit comme étant égal au rapport entre la contrainte radiale déviatorique moyenne sur la déformation radiale moyenne le long d'un axe radial:*

$$E_{palpo}^{new}(\theta) = \frac{3}{2} \frac{\left| \int_{R_i(\theta)}^{R_p(\theta)} \sigma_{rr}^{dev}(r,\theta)dr \right|}{\varepsilon(\theta)}$$

$$(2)$$

Avec $\quad \varepsilon(\theta) = \left| \int_{R_i(\theta)}^{R_p(\theta)} \varepsilon_{rr}(r,\theta)dr \right|, \quad$ où $R_i(\theta)$ and $R_p(\theta)$ sont les rayons interne et externe du domaine de palpographie.

Par ailleurs, sachant que la déformation radiale $\varepsilon_{rr}(r,\theta)$ est proportionnelle à la différence de pression $\Delta P$ (entre les deux images ayant permis d'obtenir l'image de déformation) et inversement proportionnelle à l'amplitude du module d'Young $E(r,\theta)$ on a :

$$\varepsilon_{rr}(r,\theta) = \frac{3}{2} \frac{\Delta P}{E(r,\theta)} \ h(r,\theta) \qquad\qquad (3)$$

(où la constante 3/2 a été introduite uniquement par commodité mathématique).

L'expression de la contrainte radiale déviatorique de l'équation (1) peut alors être reformulée de la manière suivante :

$$\sigma_{rr}^{dev}(r,\theta) = \Delta P \ h(r,\theta) \qquad\qquad (4)$$

Où $h(r,\theta)$ est une nouvelle fonction de forme corrective tenant compte de l'ensemble de la morphologie de la plaque, y compris les hétérogénéités géométriques de la plaque.

En tenant compte de cette nouvelle expression de la contrainte radiale déviatorique, le module de contrainte-déformation $E_{palpo}^{new}(\theta)$ devient:

$$E_{palpo}^{new}(\theta) = \frac{3}{2} \frac{\Delta P \left| \int_{R_i(\theta)}^{R_p(\theta)} h(r,\theta) \, dr \right|}{\varepsilon(\theta)}$$

$$(5)$$

Comme la fonction de forme $h(r,\theta)$ est inconnue et ne peut être mesurée directement, on estime une fonction de forme corrective approchée $h^*(r,\theta)$.

Cette fonction de forme estimée $h^*(r,\theta)$ est obtenue par une technique d'analyse par élément fini, et en considérant que la plaque est homogène, isotrope, et quasi incompressible de module d'Young E. L'analyse en élément fini a été implémentée en milieu élastique linéaire pour une différence de pression sanguine $\Delta P$. Il en résulte, qu'à partir de l'équation (3) et connaissant grâce à ce calcul aux éléments finis la distribution des déformations radiales dans l'espace $\varepsilon_{rr}^{iso}(r,\theta)$, on extrait la fonction de forme estimée $h^*(r,\theta)$ :

$$h^*(r,\theta) = \frac{2}{3} \frac{E}{\Delta P} \ \varepsilon_{rr}^{iso}(r,\theta) \qquad\qquad (6)$$

qui est utilisée pour améliorer la formule du module de contrainte-déformation devient:

$$E_{palpo}^{\text{revisited}}(\theta) = \frac{3}{2} \frac{\Delta P \left| \int_{R_i(\theta)}^{R_p(\theta)} h*(r,\theta)\, dr \right|}{\varepsilon(\theta)}$$

(7)

*Cette formule originale du module de contrainte-déformation (Equation 7) permet de retrouver le module d'Young réel d'amplitude E en considérant la plaque isotrope et homogène (i.e. $E_{palpo}^{\text{revisited}}(\theta) = E = E$), quelle que soit la géométrie de la plaque et le domaine de palpographie considéré $\Omega_{palpo}$ (avec $R_i(\theta) \leq r \leq R_p(\theta)$).*

*3. Comparaison des résultats obtenus avec le procédé selon l'invention par rapport aux résultats obtenus avec la méthode décrite dans EP 0 908 137*

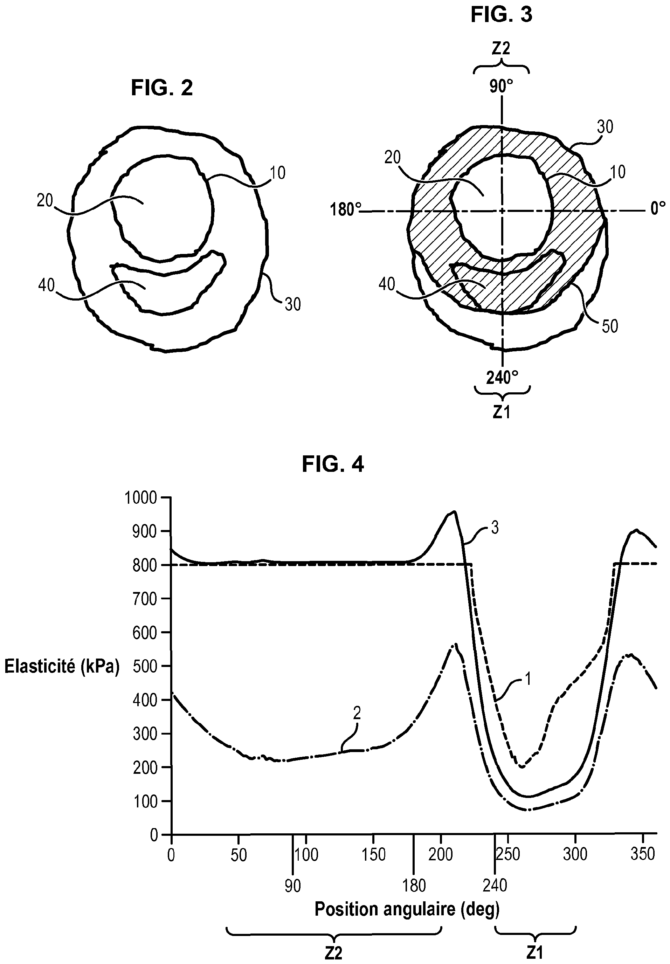

On a illustré à la figure 2 un exemple de plaque sur laquelle la méthode décrite dans EP 0 908 137 et le procédé selon l'invention ont été mis en oeuvre pour permettre une comparaison des résultats obtenus.

La plaque est une portion d'artère en coupe. Elle comprend une paroi intérieure 10 définissant une cavité 20 à l'intérieure de laquelle circule le sang, et une paroi extérieure 30 définissant la surface externe de l'artère. La plaque comprend également un espace 40 remplit d'une accumulation segmentaire de lipides.

La figure 3 illustre cette même plaque sur laquelle a été illustré le domaine de palpographie $\Omega_{palpo}$ limité par la paroi intérieure 10 et la paroi intra pariétale 50 considérée. Notons que l'espace 40 est dans cet exemple inclut dans le domaine de palpographie. Un repère orthonormé est également représenté pour permettre une mise en correspondance des positions angulaires sur la plaque avec les positions angulaires indiquées dans le graphique de la figure 4

La figure 4 est un graphique représentant une élasticité en fonction de la position spatiale angulaire ($0° \leq \theta \leq 360°$). Trois courbes sont illustrées :

- une première courbe 1 correspond à l'élasticité réelle de la plaque,
- une deuxième courbe 2 correspond à l'élasticité de la plaque calculée en utilisant la méthode décrite dans EP 0 908 137,
- une troisième courbe 3 illustre l'élasticité de la plaque calculée en mettant en oeuvre le procédé selon l'invention.

Comme on peut le constater les valeurs d'élasticité calculées représentées sur la deuxième courbe sont très différentes des valeurs d'élasticité réelles représentées sur la première courbe.

Par ailleurs en utilisant la méthode décrite dans EP 0 908 137, deux zones molles sont détectées, comme représenté sur la deuxième courbe :

- une première zone molle Z1 correspond à la partie inférieure de l'artère dans laquelle est localisée l'espace 40,
- une deuxième zone molle Z2 correspond à la partie supérieure de l'artère.

Ainsi, la méthode décrite dans EP 0 908 137 semble indiquer à l'utilisateur deux zones potentiellement à risque, alors que la zone supérieure ne présente aucun risque et correspond simplement à une zone moins épaisse de l'artère.

Ceci tient au fait que les hypothèses utilisées dans la méthode de EP 0 908 137 (i.e. parois interne et externe de la plaque cylindriques et concentriques) ne permettent pas de distinguer :

- une zone molle dans l'artère,
- d'une zone fine dans l'artère.

Ainsi, la méthode de EP 0 908 137 induit la détection d'un grand nombre de faux négatifs tendant à rendre l'information fournie à l'utilisateur inexploitable.

Au contraire et comme l'illustre la troisième courbe, les élasticités calculées avec le procédé selon l'invention sont proches de des élasticités réelles de la plaque. Par ailleurs, aucun faux négatif n'est détecté : seule la première zone molle Z1 induit une variation de l'élasticité.

Le procédé décrit ci-dessus permet donc de fournir une information temps réel exploitable à l'utilisateur lui permettant de prédire les risques de rupture d'une plaque d'athérome.

**Revendications**

1. Procédé de traitement d'image permettant la formation d'une image d'élasticité d'un corps incluant une cavité en fonction du ou des matériaux constituant ledit corps, **caractérisé en ce que** le procédé comprend les étapes consistant à :

   - Recevoir (100) une image de déformation illustrant un champ de déplacement des points du corps en fonction d'une différence de pression dans le corps,
   - Estimer (200) une fonction de forme du corps à partir de l'image de déformation, ladite étape d'estimation de la fonction de forme comprenant :

      - la détection des contours intérieur et extérieur du corps à partir de l'image de déformation pour obtenir une image de contour,
      - l'attribution d'une distribution homogène de l'élasticité dans l'image de contour pour obtenir une image de travail,
      - la détermination de la fonction de forme à partir de l'image de travail en mettant en oeuvre une analyse par éléments finis,

   - Calculer (300) une image d'élasticité du corps en fonction de la fonction de forme estimée, de la différence de pression et de l'image de déformation, ladite étape consistant à calculer une image d'élasticité comprend la résolution de l'équation suivante :

$$E_{palpo}^{\text{revisited}}(\theta) = \frac{3}{2} \frac{\left| \int_{R_i(\theta)}^{R_p(\theta)} h*(r,\theta)\, dr \right|}{\left| \int_{R_i(\theta)}^{R_p(\theta)} \varepsilon_{rr}(r,\theta)\, dr \right|} \Delta P$$

   Avec :

      ∘ $\theta$, la position angulaire dans le corps,
      ∘ r, la position radiale dans le corps,
      ∘ $\Delta P$ la différence de pression
      ∘ $R_i(\theta)$ et $R_p(\theta)$ les rayons interne et externe d'un domaine de palpographie en fonction de la position angulaire dans le corps
      ∘ $h*(r,\theta)$ la fonction de forme estimée du corps en fonction des positions radiale et angulaire dans le corps,
      ∘ $\varepsilon_{rr}(r,\theta)$ est la déformation radiale réelle du corps en fonction de la position dans le corps.

2. Procédé selon la revendication précédente, dans lequel l'étape de calcul d'une image d'élasticité consiste à calculer une image d'élasticité projetée sur la paroi interne du corps pour obtenir une image d'élasticité projetée du corps.

3. Procédé selon la revendication précédente, lequel comprend en outre une étape consistant à superposer l'image d'élasticité projetée du corps sur une image du corps acquise en utilisant un dispositif ultrasonore.

4. Procédé selon l'une des revendications précédentes, lequel comprend en outre une étape consistant à recevoir un domaine de palpographie correspondant à la sélection par un utilisateur d'une zone du corps que l'utilisateur souhaite étudier, l'étape consistant à calculer une image d'élasticité du corps étant mise en oeuvre sur le domaine de palpographie.

5. Produit programme d'ordinateur comportant un code programme enregistré sur un support de données lisible par un ordinateur pour exécuter le procédé selon l'une des revendications 1 à 4 lorsque le programme d'ordinateur est appliqué à un ordinateur pour être exécuté.

**Patentansprüche**

1. Bildbearbeitungsverfahren, das die Bildung eines Elastizitätsbildes eines Körpers zulässt, der einen Hohlraum in Abhängigkeit von dem oder den Materialien einschließt, das / die den genannten Körper bildet / bilden, **dadurch gekennzeichnet, dass** das Verfahren die Schritte umfasst, bestehend in:

   - dem Empfang (100) eines Verformungsbildes, das ein Verschiebungsfeld der Punkte des Körpers in Abhängigkeit von einer Druckdifferenz in dem Körper darstellt,
   - der Schätzung (200) einer Formfunktion des Körpers ausgehend von dem Verformungsbild, wobei der genannte Schätzungsschritt der Formfunktion umfasst:

     - die Detektion der inneren und äußeren Konturen des Körpers ausgehend von dem Verformungsbild, um ein Konturbild zu erhalten,
     - die Zuteilung einer homogenen Verteilung der Elastizität in dem Konturbild, um ein Arbeitsbild zu erhalten,
     - die Bestimmung der Formfunktion ausgehend von dem Arbeitsbild durch die Umsetzung einer Analyse durch abgeschlossene Elemente,

   - der Berechnung (300) eines Elastizitätsbildes des Körpers in Abhängigkeit von der geschätzten Formfunktion, der Druckdifferenz und des Verformungsbildes, wobei der genannte Schritt, der in der Berechnung eines Elastizitätsbildes besteht, die Lösung der folgenden Gleichung umfasst:

$$E_{palpo}^{\text{revisited}}(\theta) = \frac{3}{2} \frac{\left| \int_{R_i(\theta)}^{R_p(\theta)} h*(r,\theta)\, dr \right|}{\left| \int_{R_i(\theta)}^{R_p(\theta)} \varepsilon_{rr}(r,\theta)\, dr \right|} \Delta P$$

   in der:

     ∘ $\theta$ die Winkelposition in dem Körper ist,
     ∘ r die radiale Position in dem Körper ist,
     ∘ $\Delta P$ die Druckdifferenz ist
     ∘ $r_i(\theta)$ und $R_p(\theta)$ die internen und externen Radien eines Palpografiebereichs in Abhängigkeit von der Winkelposition im Körper sind,
     ∘ $h*(r, \theta)$ die geschätzte Formfunktion des Körpers in Abhängigkeit von den radialen und Winkelpositionen im Körper ist,
     ∘ $\varepsilon_{rr}(r, \theta)$ die reale radiale Verformung des Körpers in Abhängigkeit von der Position im Körper ist.

2. Verfahren gemäß dem voranstehenden Anspruch, bei dem der Berechnungsschritt eines Elastizitätsbildes in der Berechnung eines Elastizitätsbildes besteht, das auf die Innenwand des Körpers projiziert wird, um ein projiziertes Elastizitätsbild des Körpers zu erhalten.

3. Verfahren gemäß dem voranstehenden Anspruch, der darüber hinaus einen Schritt umfasst, bestehend in der Überlagerung des projizierten Elastizitätsbildes des Körpers auf ein Bild des Körpers, das unter Verwendung einer Ultraschallvorrichtung erworben wurde.

4. Verfahren gemäß einem der voranstehenden Ansprüche, das darüber hinaus einen Schritt umfasst, bestehend in dem Empfang eines Palpografiebereichs, der der Selektion eines Bereichs des Körpers, den der Nutzer untersuchen möchte, durch einen Nutzer entspricht, wobei der Schritt in der Berechnung eines Elastizitätsbildes des Körpers besteht, das in dem Palpografiebereich umgesetzt wird.

5. Computerprogrammprodukt, umfassend einen Programmcode, der auf einem Datenträger gespeichert ist, welcher von einem Computer lesbar ist, um das Verfahren gemäß einem der Ansprüche 1 bis 4 auszuführen, wenn das Computerprogramm auf einen Computer zur Ausführung angewendet wird.

**Claims**

1. An imaging processing method which enables an elasticity image of a body including a cavity to be produced on the basis of the material(s) forming said body, **characterised in that** the method includes the steps of:

   - receiving (100) a deformation image illustrating a field of movement of the points of the body on the basis of a pressure difference in the body,
   - estimating (200) a shape function of the body from the deformation image, said step of estimating the shape function comprising:

     - detecting the inside and outside contours of the body from the deformation image to obtain a contour image,
     - assigning a homogeneous distribution of elasticity in the contour image to obtain a work image,
     - determining the shape function from the work image by implementing a finite element analysis,

   - calculating (300) an elasticity image of the body on the basis of the estimated shape function , of the pressure difference, and of the deformation image, said step of calculating an elasticity image comprises resolving the following equation:

$$E_{palpo}^{\text{revisited}}(\theta) = \frac{3}{2} \frac{\left| \int_{R_i(\theta)}^{R_p(\theta)} h*(r,\theta)\, dr \right|}{\left| \int_{R_i(\theta)}^{R_p(\theta)} \varepsilon_{rr}(r,\theta)\, dr \right|} \Delta P$$

   with:

     ◦ $\theta$, the angular position in the body,
     ◦ r, the radial position in the body,
     ◦ $\Delta P$ the pressure difference,
     ◦ $R_i(\theta)$ and $R_p(\theta)$ the inner and outer radii of a palpography field on the basis of the angular position in the body,
     ◦ $h*(r,\theta)$ the estimated shape function of the body on the basis of the radial and angular positions in the body,
     ◦ $\varepsilon_{rr}(r,\theta)$ the real radial deformation of the body on the basis of the position in the body.

2. The method according to the preceding claim, wherein the step of calculating an elasticity image consists in calculating an elasticity image projected on the inner wall of the body to obtain a projected elasticity image of the body.

3. The method according to the preceding claim, further comprising a step of superimposing the projected elasticity image of the body on an image of the body acquired by using an ultrasonic device.

4. The method according to one of the preceding claims, further comprising a step of receiving a palpography field corresponding to the selection by a user of an area of the body that the user wants to study, the step of calculating an elasticity image of the body being implemented on the palpography field.

5. A computer programme product including a program code recorded on a computer-readable data medium to execute the method according to one of claims 1 to 4, when the computer programme is applied to a computer in order to be executed.

## FIG. 1

| 2+n<br>IMAGES ACQUISES | | 2+n<br>PRESSIONS MESUREES |
| --- | --- | --- |

| CALCUL<br>IMAGE DE DEFORMATION | | CALCUL<br>DIFFERENCE DE PRESSION |
| --- | --- | --- |

100                    200                    100

**CALCUL FONCTION DE FORME**

Détection contour
de l'image de déformation

Prise en compte
de la différence de pression

**CALCUL IMAGE D'ELASTICITE PROJETEE** — 300

**SUPERPOSITION IMAGE ACQUISE**

**FIG. 3**

**FIG. 2**

**FIG. 4**

Elasticité (kPa)

Position angulaire (deg)

## RÉFÉRENCES CITÉES DANS LA DESCRIPTION

**Documents brevets cités dans la description**

- EP 0908137 A

- EP 0908137 I